# EUROPEAN PATENT SPECIFICATION

(11) **EP 2 084 287 B1**
(45) Date of publication and mention of the grant of the patent: **30.05.2012**
(21) Application number: 07821988.8
(22) Date of filing: 29.10.2007
(51) Int. Cl.: C12P 7/16, C12N 1/15

(54) **Process for the biological production of n-butanol with high yield**
Verfahren zur biologischen Produktion von n-Butanol mit hoher Ausbeute
Procédé de production biologique de n-butanol à haut rendement

(30) Priority: 31.10.2006 WO PCT/EP2006/067993
(43) Date of publication of application: 05.08.2009
(73) Proprietor: Metabolic Explorer, 63360 Saint Beauzire (FR)
(72) Inventor: SOUCAILLE, Philippe, F-31450 Deyme (FR)
(74) Representative: Tetaz, Franck Claude Edouard
(86) International application number: PCT/EP2007/061634
(87) International publication number: WO 2008/052973

(56) References cited:
- US-A1- 2005 089 979
- GREEN EDWARD M ET AL: "GENETIC MANIPULATION OF ACID FORMATION PATHWAYS BY GENE ACTIVATION IN CLOSTRIDIUM ACETOBUTYLICUM ATCC 824" MICROBIOLOGY, SOCIETY FOR GENERAL MICROBIOLOGY, READING, GB, vol. 142, no. 8, 1996, pages 2079-2086, XP001248643 ISSN: 1350-0872 cited in the application
- HARRIS LATONIA M ET AL: "Characterization of recombinant strains of the Clostridium acetobutylicum butyrate kinase inactivation mutant: Need for new phenomenological models for solventogenesis and butanol inhibition?" BIOTECHNOLOGY AND BIOENGINEERING, vol. 67, no. 1, 5 January 2000 (2000-01-05), pages 1-11, XP002443069 ISSN: 0006-3592 cited in the application
- SONI B K ET AL: "CONTINUOUS ACETONE-BUTANOL FERMENTATION INFLUENCE OF VITAMINS ON THE METABOLIC ACTIVITY OF CLOSTRIDIUM-ACETOBUTYLICUM" APPLIED MICROBIOLOGY AND BIOTECHNOLOGY, vol. 27, no. 1, 1987, pages 1-5, XP008081386 ISSN: 0175-7598 cited in the application
- TUMMALA SESHU B ET AL: "Transcriptional analysis of product-concentration driven changes in cellular programs of recombinant Clostridium acetobutylicum strains." BIOTECHNOLOGY AND BIOENGINEERING, vol. 84, no. 7, 30 December 2003 (2003-12-30), pages 842-854, XP002443071 ISSN: 0006-3592
- TUMMALA SESHU B ET AL: "Design of antisense RNA constructs for downregulation of the acetone formation pathway of Clostridium acetobutylicum." JOURNAL OF BACTERIOLOGY, vol. 185, no. 6, March 2003 (2003-03), pages 1923-1934, XP002443072 ISSN: 0021-9193
- DESAI RUCHIR P ET AL: "Antisense RNA strategies for metabolic engineering of Clostridium acetobutylicum" APPLIED AND ENVIRONMENTAL MICROBIOLOGY, vol. 65, no. 3, March 1999 (1999-03), pages 936-945, XP002443073 ISSN: 0099-2240
- WOODS D R: "The genetic engineering of microbial solvent production" TRENDS IN BIOTECHNOLOGY, ELSEVIER, AMSTERDAM, NL, vol. 13, no. 7, July 1995 (1995-07), pages 259-264, XP004207180 ISSN: 0167-7799

### SEQUENCE LISTING

<110> METABOLIC EXPLORER
<120> PROCESS FOR THE BIOLOGICAL PRODUCTION OF N BUTANOL WITH HIGH YIELD
<130> 351965 D24755
<150> PCT/EP2006/067993
   <151> 2006-10-31
<160> 30
<170> PatentIn version 3.3
<210> 1
   <211> 36
   <212> DNA
   <213> Artificial
<220>
   <223> PCR primer
<400> 1
   aaaaggatcc tagtaaaagg gagtgtacga ccagtg 36
<210> 2
   <211> 57
   <212> DNA
   <213> Artificial
<220>
   <223> PCR primer
<400> 2
   ggggtcgcga aaaaaggggg gattattagt aatctataca tgttaacatt cctccac 57
<210> 3
   <211> 46
   <212> DNA
   <213> Artificial
<220>
   <223> PCR primer
<400> 3
   cccccttttt tcgcgacccc acttcttgca cttgcagaag gtggac 46
<210> 4
   <211> 37
   <212> DNA
   <213> Artificial
<220>
   <223> PCR primer
<400> 4
   aaaaggatcc tctaaattct gcaatatatg ccccccc 37
<210> 5
   <211> 28
   <212> DNA
   <213> Artificial
<220>
   <223> PCR primer
<400> 5
   ataacaggat atatgctctc tgacgcgg 28
<210> 6
   <211> 28
   <212> DNA
   <213> Artificial
<220>
   <223> PCR primer
<400> 6
   gatcatcact cattttaaac atggggcc 28
<210> 7
   <211> 41
   <212> DNA
   <213> Artificial
<220>
   <223> PCR primer
<400> 7
   aaaaggatcc cagacactat aatagcttta ggtggtaccc c 41
<210> 8
   <211> 58
   <212> DNA
   <213> Artificial
<220>
   <223> PCR primer
<400> 8
   ggggaggcct aaaaaggggg attataaaaa gtagttgaaa tatgaaggtt taaggttg 58
<210> 9
   <211> 48
   <212> DNA
   <213> Artificial
<220>
   <223> PCR primer
<400> 9
   cccccttttt aggcctcccc atatccaatg aacttagacc catggctg 48
<210> 10
   <211> 49
   <212> DNA
   <213> Artificial
<220>
   <223> PCR primer
<400> 10
   aaaaggatcc gtgttataat gtaaatataa ataaatagga ctagaggcg 49
<210> 11
   <211> 28
   <212> DNA
   <213> Artificial
<220>
   <223> PCR primer
<400> 11
   taccaccttc tttcacgctt ggctgcgg 28
<210> 12
   <211> 28
   <212> DNA
   <213> Artificial
<220>
   <223> PCR primer
<400> 12
   tatttaaaga ggcattatca ccagagcg 28
<210> 13
   <211> 39
   <212> DNA
   <213> Artificial
<220>
   <223> PCR primer
<400> 13
   aaaaggatcc gctttaaaat ttggaaagag gaagttgtg 39
<210> 14
   <211> 56
   <212> DNA
   <213> Artificial
<220>
   <223> PCR primer
<400> 14
   ggggaggcct aaaaaggggg ttagaaatct ttaaaaattt ctctatagag cccatc 56
<210> 15
   <211> 57
   <212> DNA
   <213> Artificial
<220>
   <223> PCR primer
<400> 15
   cccccttttt aggcctcccc ggtaaaagac ctaaactcca agggtggagg ctaggtc 57
<210> 16
   <211> 48
   <212> DNA
   <213> Artificial
<220>
   <223> PCR primer
<400> 16
   aaaaggatcc cccattgtgg agaatattcc aaagaagaaa ataattgc 48
<210> 17
   <211> 31
   <212> DNA
   <213> Artificial
<220>
   <223> PCR primer
<400> 17
   cagaaggcaa gaatgtatta agcggaaatg c 31
<210> 18
   <211> 31
   <212> DNA
   <213> Artificial
<220>
   <223> PCR primer
<400> 18
   cttcccatta tagctcttat tcacattaag c 31
<210> 19
   <211> 43
   <212> DNA
   <213> Artificial
<220>
   <223> PCR primer
<400> 19
   aaaaggatcc tattataaca gtcaaaccca ataaaatact ggg 43
<210> 20
   <211> 54
   <212> DNA
   <213> Artificial
<220>
   <223> PCR primer
<400> 20
   ggggaggcct aaaaaggggg ttaatccatt tgtattttct cccttcataa tgcc 54
<210> 21
   <211> 57
   <212> DNA
   <213> Artificial
<220>
   <223> PCR primer
<400> 21
   cccccttttt aggcctcccc tttattttgc atgcttatat aataaattat ggctgcg 57
<210> 22
   <211> 40
   <212> DNA
   <213> Artificial
<220>
   <223> PCR primer
<400> 22
   aaaaggatcc gcttttcctt cttttacaag atttaaagcc 40
<210> 23
   <211> 26
   <212> DNA
   <213> Artificial
<220>
   <223> PCR primer
<400> 23
   cacttttatt tatcaagctg taggcc 26
<210> 24
   <211> 25
   <212> DNA
   <213> Artificial
<220>
   <223> PCR primer
<400> 24
   tatacctttt gaacctagga aaggc 25
<210> 25
   <211> 43
   <212> DNA
   <213> Artificial
<220>
   <223> PCR primer
<400> 25
   aaaaggatcc gcctcttctg tattatgcaa ggaaagcagc tgc 43
<210> 26
   <211> 59
   <212> DNA
   <213> Artificial
<220>
   <223> PCR primer
<400> 26
   ggggaggcct aaaaaggggg tatataaaat aaatgtgcct taacatctaa gttgaggcc 59
<210> 27
   <211> 85
   <212> DNA
   <213> Artificial
<220>
   <223> PCR primer
<400> 27
   cccccttttt aggcctcccc gtttatcctc ccaaaatgta aaatataatt aaaatatatt 60
   aataaacttc gattaataaa cttcg 85
<210> 28
   <211> 43
   <212> DNA
   <213> Artificial
<220>
   <223> PCR primer
<400> 28
   aaaaggatcc ccttttagcg tataaagttt tatatagcta ttg 43
<210> 29
   <211> 33
   <212> DNA
   <213> Artificial
<220>
   <223> PCR primer
<400> 29
   catgttctat tgttactatg gaagaggtag tag 33
<210> 30
   <211> 29
   <212> DNA
   <213> Artificial
<220>
   <223> PCR primer
<400> 30
   gcagttatta taaatgctgc tactagagc 29

## Description

### FIELD OF INVENTION

The invention comprises a process for the bioconversion of a fermentable carbon source to n-butanol at high yield by a metabolically engineered microorganism.

### BACKGROUND OF THE INVENTION

n-Butanol is a colorless, neutral liquid of medium volatility with restricted miscibility (about 7-8%) in water, but freely miscible with all common solvents such as glycols, ketones, alcohol, aldehydes, ethers, and aromatic and aliphatic hydrocarbons n-Butanol is used i) to make other chemicals, ii) as a solvent and iii) as an ingredient in formulated products such as cosmetics. The major uses of n-butanol as a feed-stock are in the synthesis of acrylate/methacrylate esters, glycol ethers, n-Butyl acetate, amino resins and n-Butylamines. Currently more than 9 millions tons of n-Butanol are consumed annually in the world.

More recently it has been shown that n-butanol is a better biofuel than ethanol due to lower vapour pressure, higher energy content (closer to that of gasoline) and lesser susceptibility to separation in the presence of water. Furthermore, n-butanol can be blended at higher concentrations than ethanol for use in standard vehicle engines and it does not require automakers to compromise on performance to meet environmental regulations; it is also suitable for transport in pipelines and as a result it has the potential to be introduced into gasoline quickly and avoid the need for additional large-scale supply infrastructures.

n-butanol can be produced as an acetone/n-butanol/ethanol (ABE) mixture by the fermentation of carbohydrate by solventogenic *Clostridia.* The ABE fermentations are biphasic. During the first acidogenic phase, high growth rate is accompanied by acetic and butyric acids production. In the second solventogenic phase growth rate decrease and the solvents (ABE) are produced with the concomitant consumption of the organic acids produced in the first phase. Carbon dioxide and hydrogen are produced throughout the fermentation.

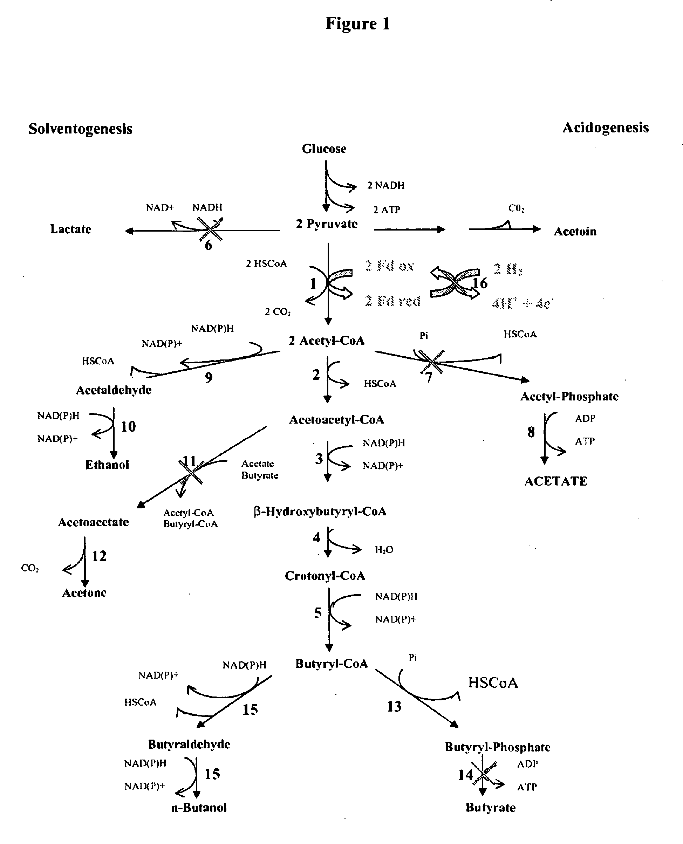

The biological production of n-butanol, presented in figure 1, requires the formation of butyryl-CoA as an intermediate which can be reduced, depending on the physiological conditions, by two different bi-functional aldehyde-alcohol dehydrogenases encoded by *adhE1* and *adhE2.* Butyryl-CoA can also be converted to butyric acid by a phospho-transbutyrylase and a butyrate kinase encoded respectively by the *ptb* and *buk* genes. Acetone is produced from aceto-acetyl-CoA (an intermediate in the production of butyryl-CoA) by a CoA-transferase and an acetoacetate decarboxylase encoded respectively by the *crfAB* and *adc* genes. Hydrogen is produced by an iron only hydrogenase encoded by the *hydA* gene. When cultures are performed in the presence of carbon monoxide, a hydrogenase inhibitor, n-butanol, ethanol and lactate are the main fermentation products. Lactate is produced from pyruvate by a lactate dehydrogenase encoded by the *ldh* gene.

*Clostridium acetobutylicum* strains with an inactivated *buk* gene (obtained by single crossing over with a non-replicable plasmid) have already been described in the article (Green et al., 1996). The non-replicable vector pJC4BK, with a 0.8 kb internal buk fragment was integrated into the chromosomal buk gene which leaded to an inactivation of the endogenous gene. The obtained strain was named "mutant PJC4BK"from the name of the plasmid. As precised in this article, this gene integration did not completely eliminate enzyme activity nor butyrate formation due to the instability of this type of gene inactivation that can reverse to wild type by plasmid excision. This mutant strain was then used in several studies (Green and Bennett, 1998; Desai and Harris, 1999; Harris et al., 2000).

Traditionally, the commercial ABE fermentation was conducted only in a batch mode due to continuous cultures instability of the producing *Clostridia.* Several solvent yielding fermentation processes have been described. These processes yield n-butanol, acetone and ethanol in a ratio of 6:3:1. Solvent yields of 29-34% (18-25% for n-butanol only) of fermentable carbon source have been reported in the literature. A total solvent concentration of 16-24 g/l and a n-butanol concentration of 10-14 g/l is generally the limit due to toxicity of n-butanol produced. However, these low titers of solvent no longer seem to be an economical limitation to the process as it has recently been demonstrated that solvents can be recovered during fermentation by the use of the "low cost" gas striping technology.

The problem to be solved by the present invention is to obtain a stable mutant strain with no butyrate kinase activity, that could be cultured for several generations without any possibility of reversion to the wild type genotype. This strain would be useful for the biological production of n-butanol at high yield, from an inexpensive carbon substrate such as glucose or other sugars, by genetically stable cultures of *Clostridia.* The number of biochemical steps to inactivate and the complexity of the regulation of the metabolism necessitate, for an industrial feasible process of n-butanol production, the use of a metabolically engineered whole cell catalyst.

### SUMMARY OF THE INVENTION

Applicants have solved the stated problem and the present invention provides a method for bioconverting a fermentable carbon source to n-butanol as a major product by genetically stable cultures of *Clostridia.*

The invention provides for a method for the production of n-butanot by culturing a microorganism of a *Clostridia* species in an appropriate culture medium comprising a source of carbon and recovery of n-butanol from the culture medium, wherein the gene buk involved in butyrate formation is deleted in the microorganism, the deletion consisting in the removal of at least 50% of the coding sequence of the deleted gene.

The invention also provides for a microorganism of a *Clostridia* species wherein at **the gene ptb** involved in butyrate formation is **furthermore** deleted in the microorganism, the deletion consisting in the removal of at least 50% of the coding sequence of the deleted gene.

Glucose is used as a model substrate and recombinant *Clostridium acetobutylicum* is used as the model host. In this invention, a stable recombinant *C. acetobutylicum* unable to metabolize butyryl-CoA to butyrate is constructed by deleting the gene coding for the butyrate kinase *(buk).* In a further aspect of this invention a recombinant *C*. *acetobutylicum* unable to produce acetone is constructed by additionally deleting the genes coding for the CoA-transferase *(ctfAB).* In a further aspect of this invention a recombinant strain unable to produce lactate is constructed by additionally deleting the gene coding for the lactate dehydrogenase *(ldh).* Furthermore, a recombinant *C*. *acetobutylicum* unable to produce acetate is constructed by additionally deleting the genes coding for the phosphotransacetylase and/or acetate kinase *(pta* and *ack).*

The present invention may be generally applied to include any carbon substrate that is readily converted to acetyl-coA.

Accordingly it is an object of the present invention to provide a recombinant organism, useful For the production of n-butanol comprising: (a) at least deletion of the **buk** genes involved in the conversion of butyryl-CoA to butyrate and (b) at least deletion of one of the two genes encoding the CoA-transferase activity. Optionally the recombinant organism may comprise i) inactivating mutations in endogenous genes selected from the group consisting of: (a) a gene encoding a polypeptide having lactate dehydrogenase activity and (b) a gene encoding a polypeptide having phospho-transacetylase or actate kinase activity.

In another embodiment the invention provides a stable process for the production of n-butanol at high yield from a recombinant organism comprising: (a) contacting the recombinant organism of the present invention with at least one carbon source selected from the group consisting of monosacchurides, oligosaccharides, polysaccharides, and single-carbon substrates whereby n-butanol is produced; optionally (b) recovering then-butanol during the production through a step of gas striping and (c) purifying n-butanol from the condensate by distillation.

### BRIEF DESCRIPTION OF THE DRAWINGS

The accompanying drawing which is incorporated in and constitutes a part of this specification exemplifies the invention and together with the description, serve to explain the principles of this invention.

**Figure 1** depicts the genetic engineering of central metabolism in the development of a butanol production system from carbohydrates.
1 : Pyruvate-ferredoxin oxydoreductase ; 2 : Thiolase ; 3 : β-Hydroxybutyryl-CoA dehydrogenase ; 4 : Crotonase ; 5 : Butyryl-CoA dehydrogenase ; 6 : Lactate dehydrogenase ; 7 : Phospho-transacetylase ; 8 : Acetate kinase ; 9 : Acetaldehyde deshydrogenase ; 10 : Ethanol dehydrogenase ; 11 : CoA transferase (Acetoacetyl-CoA :acetate/butyrate : CoA transferase) ; 12 : Acetoacetate decarboxylase ; 13 : Phospho-transbutyrylase ; 14 : Butyrate kinase ; 15 : Butyraldehyde-Butanol dehydrogenase ; 16 : hydrogenase.

### DETAILED DESCRIPTION OF THE INVENTION

As used herein the following terms may be used for interpretation of the claims and specification.

The term "microorganism" refers to all kind of unicellular organisms, including prokaryotic organisms like bacteria, and eukaryotic organisms like yeasts.

The expression "appropriate culture medium" refers to a culture medium adapted for the used microorganism as it is well known by the man skilled in the art.

The term "carbon substrate" or "source of carbon" means any carbon source capable of being metabolized by a microorganism wherein the substrate contains at least one carbon atom. Authors refer particularly to renewable, inexpensive and fermentable carbon sources such as monosaccharides, oligosaccharides, polysaccharides, single-carbon substrates, and polyols such as glycerol. Single carbon substrate are defined as carbon molecules that contain only one carbon atom such as methanol.

Monosaccharides of the formula (CH₂O)ₙ are also called oses or "simple sugars"; monosaccharides include saccharose, fructose, glucose, galactose and mannose.

Other carbon sources comprising more than one monosaccharide are called disaccharides, trisaccharides, oligosaccharides and polysaccharides. Disaccharides include saccharose (sucrose), lactose and maltose. Starch and hemicellulose are polysaccharides, also know as "complex sugars".

Therefore, the term "source of carbon" means any product cited above, and mixture thereof.

The term "attenuation" refers to a decreased expression of a gene or a decreased activity of the protein, product of the gene. The man skilled In the art knows numerous means to obtain this result, and for example:
- Introduction of a mutation into the gene, decreasing the expression level of this gene, or the level of activity of the encoded protein.
- Replacement of the natural promoter of the gene by a low strength promoter, resulting in a lower expression
- Use of elements destabilizing the corresponding messenger RNA or the protein
- Deletion of the gene if no expression is needed.

The term "deleted gene" means that at least 50% of the coding sequence was removed, and preferably at least 80%.

In the description of the present invention, enzymes are identified by their specific activities. This definition thus includes all polypeptides that have the defined specific activity also present in other organisms, more particularly in other microorganisms. Often enzymes with similar activities can be identified by their grouping to certain families defined as PFAM or COG.

PFAM (protein families database of alignments and hidden Markov models; http://www.sanger.ac.uk/software/Pfam/) represents a large collection of protein sequence alignments. Each PFAM makes it possible to visualize multiple alignments, see protein domains, evaluate distribution among organisms, gain access to other databases, and visualize known protein structures.

COGs (clusters of orthologous groups of proteins; http://www.ncbi.nlm.nih.gov/COG/) are obtained by comparing protein sequences from 43 fully sequenced genomes representing 30 major phylogenic lines. Each COG is defined from at least three lines, which permits the identification of former conserved domains.

The means of identifying homologous sequences and their percentage homologies are well known to those skilled in the art, and include in particular the BLAST programs, which can be used from the website hqg:/twww.ncbi.niM.nih gov/BLAST/ with the default parameters indicated on that website. The sequences obtained can then be exploited (e.g., aligned) using, for example, the programs CLUSTALW (http://www.ebi.ac.uk/clustalw/) or MULTALIN (htto://prodes.toulouse.inra.fr/multaling/cgi-bin/multaling.pl), with the default parameters indicated on those websites.

Using the references given on GenBank for known genes, those skilled in the art are able to determine the equivalent genes in other organisms, bacterial strains, yeasts, fungi, mammals, plants, etc. This routine work is advantageously done using consensus sequences that can be determined by carrying out sequence alignments with genes derived from other microorganisms, and designing degenerate probes to clone the corresponding gene in another organism. These routine methods of molecular biology are well known to those skilled in the art, and are described, for example, in Sambrook et al. (Molecular Cloning: a Laboratory Manual. 2nd ed. Cold Spring Harbor Lab., Cold Spring Harbor, New York, 1989.).

The present invention provides a method for the fermentative batch or continuous production of n-butanol by culturing a microorganism in an appropriate culture medium comprising a carbon source and the simultaneous recovery of n-butanol from the culture medium wherein at least one gene involved in butyrate formation is deleted in the microorganism.

The invention provides a method wherein the microorganism is modified to be unable to convert butyryl-CoA to butyrate due to the deletion of the gene encoding for butyrate kinase *(buk).* Deletion of genes in *Clostridia* can be done using the method recently described in patent application WO2008 040387allowing the i) replacement of the gene to delete with an erythromycin resistance gene and ii) removal of the erythromycin resistance gene with a recombinase.

In a further embodiment of the invention, the microorganism is unable to produce acetone due to an additional attenuation or a deletion of at least one of the gene encoding for CoA-transferase *(ctfAB)* or acetoacetate decarboxylase *(adc).* Deletion of one of these genes can be done using the method recently described in patent application WO2008 040387

In a further embodiment of the invention, the microorganism used in the method of the invention is unable to produce lactate. In particular this can be due to an additional deletion of the gene *ldh* encoding for lactate dehydrogenase. Deletion of *ldh* can be done using the method recently described in patent application WO 2008 040387.

In a further embodiment, the microorganism is modified in such a way to be unable to produce acetate. This result can be achieved by an additional delection of at least one of the genes encoding for phospho-transacetylase *(pta)* or acetate kinase *(ack).* Deletion of one; of these genes can be done using the method recently described in patent application WO2008 040387

An embodiment of the disclosure also provides a microorganism with a decreased flux of hydrogen production and then a redirection of the flux of reducing equivalent toward n-butanol production; this can be done by attenuating the gene encoding the hydrogenase (*hydA*), an enzyme that provides a sink for reducing equivalent in the form of hydrogen production. Attenuation of *hydA* can be done by replacing the natural promoter by a low strength promoter or by element destabilizing the corresponding messenger RNA or the protein. If needed, complete attenuation of the gene can also be achieved by a deletion of the corresponding DNA sequence.

Preferably, the used microorganism is selected among the group consisting of *C. acetobtitylicum, C. beijerinckii, C. saccharoperbutylacetonicum* or *C. saccharobutylicum.*

In another embodiment of the invention, the culture is continuous and stable.

In another embodiment, the method according to the invention comprises the following steps:
(a) contacting a microorganism with at least one carbon sources selected from the group consisting of glucose, xylose, arabinose, sucrose, monosaccharides, oligosaccharides, polysaccharides, cellulose, xylan, starch or its derivatives and glycerol, whereby n-butanol is produced
(b) Recovering the n-butanol during the fermentation by gas striping and
(c) Isolation of n-butanol from the condensate by distillation.

Those skilled in the art are able to define the culture conditions for the microorganisms according to the invention. In particular the clostridia are fermented at a temperature between 20°C and 55°C, preferentially between 25°C and 40°C, and more specifically about 35°C for *C. acetobutylicum.*

The fermentation is generally conducted in fermentors with an inorganic culture medium of known defined composition adapted to the bacteria used, containing at least one simple carbon source, and if necessary a co-substrate necessary for the production of the metabolite.

The invention is also related to the microorganism as described previously. Preferably, this microorganism is selected among the group consisting of *C. acetobutylicum, C. beijerinckii, C. saccharoperbutylacetonicum* or *C. saccharobutylicum.*

### EXAMPLE 1

### Construction of strains unable to produce butyrate: Clostridium acetobutylicum Δcac1515 Δupp Δbuk

To delete the *buk* gene, the homologous recombination strategy described by Croux & Soucaille (2006) in patent application WO 2008 040387 is used. This strategy allows the insertion of an erythromycin resistance cassette, while deleting most of the gene concerned. The *buk* deletion cassette in pCons::upp was constructed as follows.

**Table 1: primers sequences**

| Name | | Primer sequences |
|---|---|---|
| Buk 1 | SEQ ID N°1 | aaaaggatcc**tagtaaaagggagtgtacgaccagtg** |
| Buk 2 | SEQ ID N°2 | ggggtcgcgaaaaaaggggg**gattattagtaatctatacatgttcctccac** |
| Buk 3 | SEQ ID N°3 | cccccttttttcgcgacccc**acttcttgcacttgcagaaggtggac** |
| Buk 4 | SEQ ID N°4 | aaaaggatcctctaaattctgcaatatatgcccccc |
| Buk 0 | SEQ ID N°5 | ataacaggatatatgctctctgacgcgg |
| Buk 5 | SEQ ID N°6 | qatcatcactcattttaaacatqqqqcc |

Two DNA fragments surrounding *buk* were PCR amplified with the Pwo polymerase with total DNA from *C*. *acetobutylicum* as template and two specific couples of olignonucleotides. With the couples of primers BUK 1-BUK 2 and BUK 3-BUK 4, two DNA fragments were respectively obtained. Both primers BUK 1 and BUK 4 introduce a BamHI site while primers BUK 2 and BUK 3 have a complementary region which introduces a NruI site. DNA fragments BUK 1-BUK 2 and BUK 3-BUK 4 were joined in a PCR fusion experiment with primers BUK 1 and BUK 4 and the resulting fragment was cloned in pCR4-TOPO-Blunt to yield pTOPO :buk. At the unique Stul site of pTOPO :buk, an antibiotic resistance MLS gene with FRT sequences on both sides was introduced from the Stul fragment of pUC18-FRT-MLS2. The BUK deletion cassette obtained after BamHI digestion of the resulting plasmid was cloned into pCons::upp at the BamHI site to yield the pREPABUK::upp plasmid.

The pREPΔBUK::upp plasmid was used to transform by electroporation *C*. *acetobutylicum* MGCΔ*cac15Δupp* strain. After selection on Petri plate for clones resistant to erythromycin (40 µg/ml), one colony was cultured for 24 hours in liquid synthetic medium with erythromycin at 40 µg/ml and 100 µl of undiluted culture was plated on RCA with erythromycin at 40 µg/ml and 5-FU at 400 µM. Colonies resistant to both erythromycin and 5-FU were replica plated on both RCA with erythromycin at 40 µg/ml and RCA with thiamphenicol at 50 µg/ml to select clones where 5-FU resistance is also associated with thiamphenicol sensitivity. The genotype of clones resistant to erythromycin and sensitive to thiamphenicol was checked by PCR analysis (with primers BUK 0 and BUK 5 located outside of the *buk* deletion cassette). The Δ*cac15*Δ*upp*Δ*buk::mls^{R}* strain which have lost pREPΔbuk::upp was isolated.

The Δ*cac15*Δ*upp*Δ*buk::mls^{R}* strain was transformed with pCLFI.1 vector expressing the *Flp1* gene encoding the Flp recombinase from *S*. *cerevisiae.* After transformation and selection for resistance to thiamphenicol (50 µg/ml) on Petri plate, one colony was cultured on synthetic liquid medium with thiamphenicol at 50 µg/ml and appropriate dilutions were plated on RCA with thiamphenicol at 50 µg/ml. Thiamphenicol resistant clones were replica plated on both RCA with erythromycin at 40 µg/ml and RCA with thiamphenicol at 50 µg/ml. The genotype of clones with erythromycin sensitivity and thiamphenicol resistance was checked by PCR analysis with primers BUK 0 and BUK 5. Two successive 24 hours cultures of the Δ*cac15A*Δ*upp*Δ*buk* strain with erythromycin sensitivity and thiamphenicol resistance were carried out in order to lose pCLF1.1. The Δ*cac15*Δ*upp*Δ*buk* strain which has lost pCLF1.1 was isolated according to its sensitivity to both erythromycin and thiamphenicol.

### EXAMPLE 2

### Construction of strains unable to produce butyrate and acetone: C. acetobutylicum Δcac1515 Δupp Δbuk ΔctfAB

To delete the *crfAB* genes, the homologous recombination strategy described by Croux & Soucaille (2006) in patent application WO 2008 040 387 is used. This strategy allows the insertion of an erythromycin resistance cassette, while deleting most of the genes concerned. The *ctfAB* deletion cassette in pCons::upp was constructed as follows.

**Table 2 : primers scqucnccs**

| Name | | Primer sequences |
|---|---|---|
| Ctf 1 | SEQ ID N°7 | aaaaggatcc**cagococtotootogctttoggtggtocccc** |
| Ctf 2 | SEQ ID N°8 | ggggaggcctaaaaagggg**attataaaaagtagttgaaatatggttg** |
| Ctf 3 | SEQ ID N°9 | ccccctttttaggcctcccc**atatccaatgaacttagacccatggctg** |
| Ctf 4 | SEQ ID N°10 | aaaaggatcc**gtgttataatgtaaatataaataaataggactagaggcg** |
| Ctf 0 | SEQ ID N°11 | taccaccttctttcacgcttggctgcgg |
| Ctf 5 | SEQ ID N°12 | tatttaaagaggcattatcaccagagcg |

Two DNA fragments surrounding *ctfAB* were PCR amplified with the Pwo polymerase with total DNA from *C. acetobutylicum* as template and two specific couples of olignonucleotides. With the couples of primers CTF 1-CTF 2 and CTF 3-CTF 4, two DNA fragments were respectively obtained. Both primers CTF 1 and CTF 4 introduce a BamHI site while primers CTF 2 and CTF 3 have a complementary region which introduces a Stul site. DNA fragments CTF 1-CTF 2 and CTF 3-CTF 4 were joined in a PCR fusion experiment with primers CTF 1 and CTF 4 and the resulting fragment was cloned in pCR4-TOPO-Blunt to yield pTOPO :CTF. At the unique StuI site of pTOPO:CTF, an antibiotic resistance MLS gene with FRT sequences on both sides was introduced from the Stul fragment of pUC18-FRT-MLS2. The UPP deletion cassette obtained after BamHI digestion of the resulting plasmid was cloned into pCons::upp at the BamHI site to yield the pREPΔCTF::upp plasmid.

The pREPΔCTF::upp plasmid was used to transform by electroporation *C. acetobutylicum* MGC*Δcac15ΔuppΔbuk* strain. After selection on Petri plate for clones resistant to erythromycin (40 µg/ml), one colony was cultured for 24 hours in liquid synthetic medium with erythromycin at 40 µg/ml and 100 µl of undiluted culture was plated on RCA with erythromycin at 40 µg/ml and 5-FU at 400 µM. Colonies resistant to both erythromycin and 5-FU were replica plated on both RCA with erythromycin at 40 µg/ml and RCA with thiamphenicol at 50 µg/ml to select clones where 5-FU resistance is also associated with thiamphenicol sensitivity. The genotype of clones resistant to erythromycin and sensitive to thiamphenicol was checked by PCR analysis (with primers CTF 0 and CTF 5 located outside of the *ctfAB* deletion cassette). The Δ*cac15*Δ*uppdbuk* Δ*ctfAB::mls^{R}* strain which have lost pREPΔCTF::upp was isolated.

The Δ*cac15*Δ*upp*Δ*buk*Δ*ctfAB::mls^{R}* strain was transformed with pCLF1.1 vector expressing the *Flp1* gene encoding the Flp recombinase from *S*. *cerevisiae.* After transformation and selection for resistance to thiamphenicol (50 µg/ml) on Petri plate, one colony was cultured on synthetic liquid medium with thiamphenicol at 50 µg/ml and appropriate dilutions were plated on RCA with thiamphenicol at 50 µg/ml. Thiamphenicol resistant clones were replica plated on both RCA with erythromycin at 40 µg/ml and RCA with thiamphenicol at 50 µg/ml. The genotype of clones with erythromycin sensitivity and thiamphenicol resistance was checked by PCR analysis with primers CTF 0 and CTF 5. Two successive 24 hours cultures of the Δca*c15*Δu*pp*Δ*buk*Δ*ctfAB* strain with erythromycin sensitivity and thiamphenicol resistance were carried out in order to lose pCLF 1. The Δ*cac15*Δ*upp*Δ*buk*Δ*ctfAB* strain which has lost pCLF1.1 was isolated according to its sensitivity to both erythromycin and thiamphenicol.

### EXAMPLE 3

### Construction of strains unable to produce butyrate, acetone and lactate: C. acetobutylicum Δcac1515 Δupp Δbuk ΔctfAB Δldh

To delete the *ldh* gene, the homologous recombination strategy described by Croux & Soucaille (2006) in patent application WO 2008 040 387 is used. This strategy allows the insertion of an erythromycin resistance cassette, while deleting most of the genes concerned. The *ldh* deletion cassette in pCons::upp was constructed as follows.

**Table 3 : primers sequences**

| Name | | Primer sequences |
|---|---|---|
| Ldh 1 | SEQ ID N°13 | |
| Ldh 2 | SEQ ID N°14 | |
| Ldh 3 | SEQ ID N°15 | |
| Ldh 4 | SEQ ID N°16 | |
| Ldh 0 | SEQ ID N°17 | CAGAAGGCAAGAATGTATTAAGCGGAAATGC |
| Ldh 5 | SEQ ID N°18 | CTTCCCATTATAGCTCTTATTCACATTAAGC |

Two DNA fragments surrounding *ldh* (*CAC267*) were PCR amplified with the Pwo polymerase with total DNA from *C. acetobutylicum* as template and two specific couples of olignonucleotides. With the couples of primers LDH 1-LDH 2 and LDH 3-LDH 4, 1 135 bp and 1177 bp DNA fragments were respectively obtained. Both primers LDH 1 and LDH 4 introduce a BamHI site while primers LDH 2 and LDH 3 have a complementary region which introduces a StuI site. DNA fragments LDH 1-LDH 2 and LDH 3-LDH 4 were joined in a PCR fusion experiment with primers LDH I and LDH 4 and the resulting fragment was cloned in pCR4-TOPO-Blunt to yield pTOPO :LDH. At the unique StuI site of pTOPO :LDH, an antibiotic resistance MLS gene with FRT sequences on both sides was introduced from the 1372 bp StuI fragment of pUC18-FRT-MLS2. The UPP deletion cassette obtained after BamHI digestion of the resulting plasmid was cloned into pCons::upp at the BamHI site to yield the pREPOLDH::upp plasmid.

The pREPOLDH::upp plasmid was used to transform by electroporation *C*. *acetobutylicum* MGCΔ*cac15A*Δ*pp*Δ*buk*Δ*cffAB* strain. After selection on Petri plate for clones resistant to erythromycin (40 µg/ml), one colony was cultured for 24 hours in liquid synthetic medium with erythromycin at 40 µg/ml and 100 µl of undiluted culture was plated on RCA with erythromycin at 40 µg/ml and 5-FU at 400 µM. Colonies resistant to both erythromycin and 5-FU were replica plated on both RCA with erythromycin at 40 µg/ml and RCA with thiamphenicol at 50 µg/ml to select clones where 5-FU resistance is also associated with thiamphenicol sensitivity. The genotype of clones resistant to erythromycin and sensitive to thiamphenicol was checked by PCR analysis (with primers LDH 0 and LDH 5 located outside of the *ldh* deletion cassette). The Δ*cac15*Δ*upp*Δ*buk* Δ*ctfAB* Δ*ldh::mls^{R}* strain which have lost pREPOLDH::upp was isolated.

The Δ*cac15*Δ*upp*Δ*buk*Δ*ctf:AB*Δ*ldh::mls^{R}* strain was transformed with pCLF 1.1 vector expressing the *Flp1* gene encoding the Flp recombinase from *S*. *cerevisiae.* After transformation and selection for resistance to thiamphenicol (50 µg/ml) on Petri plate, one colony was cultured on synthetic liquid medium with thiamphenicol at 50 µg/ml and appropriate dilutions were plated on RCA with thiamphenicol at 50 µg/ml. Thiamphenicol resistant clones were replica plated on both RCA with erythromycin at 40 µg/ml and RCA with thiamphenicol at 50 µg/ml. The genotype of clones with erythromycin sensitivity and thiamphenicol resistance was checked by PCR analysis with primers LDH 0 and LDH 5. Two successive 24 hours cultures of the *Δcac15ΔuppΔbukΔctfAB Δldh* strain with erythromycin sensitivity and thiamphenicol resistance were carried out in order to lose pCLF1.1. The *Δcac15 Δupp ΔbukΔctfABΔldh* strain which has lost pCLF1 was isolated according to its sensitivity to both erythromycin and thiamphenicol.

### EXAMPLE 4

### Construction of strains unable to produce butyrate, acetone, lactate and acetate: C. acetobutylicum Δcac1515 Δupp Δbuk ΔctfAB Δldh Δpta-ack

To delete the *pta* and *ack* genes, the homologous recombination strategy described by Croux & Soucaille (2006) in patent application WO 2008 040387 is used. This strategy allows the insertion of an erythromycin resistance cassette, while deleting most of the genes concerned. The *pta-ack* deletion cassette in pCons::upp was constructed as follows.

**Table 4 : primers sequences**

| Name | | Primer sequences |
|---|---|---|
| PA 1 | SEQ ID N°19 | aaaaggatcctattataacagtcaaacccaotaaantactggg |
| PA 2 | SEQ ID N°20 | ggggaggcctaaaaagggggttaatccatttgtawttctcccttcataatgcc |
| PA 3 | SEQ ID N°21 | ccccctttttaggcctcccctttnttttgcntgcttatntaatnanttntggctgcg |
| PA 4 | SEQ ID N°22 | aaaaggatccgcttttccttcttttacaagatttaaagcc |
| PA 0 | SEQ ID N°23 | cacttttatttatcaagctgtaggcc |
| PA 5 | SEQ ID N°24 | tataccttttgaacctaggaaaggc |

Two DNA fragments surrounding *pta-ack* were PCR amplified with the Pwo polymerase with total DNA from *C*. *acetobutylicum* as template and two specific couples of olignonucleotides. With the couples of primers PA I-PA.2 and PA 3-PA 4, two DNA fragments were respectively obtained. Both primers PA I and PA 4 introduce a BamHI site while primers PA 2 and PA 3 have a complementary region which introduces a Stul site. DNA fragments PA I-PA 2 and PA 3···PA 4 were joined in a PCR fusion experiment with primers PA 1 and PA 4 and the resulting fragment was cloned in pCR4-TOPO-Blunt to yield pTOPO :PA. At the unique Stul site of pTOPO :PA, an antibiotic resistance MLS gene with FRT sequences on both sides was introduced from the Stul fragment of pUC18-FRT-MLS2. The UPP deletion cassette obtained after BamHI digestion of the resulting plasmid was cloned into pCons::upp at the BamHI site to yield the pREPΔPA::upp plasmid.

The pREPΔPA::upp plasmid was used to transform by electroporation *C*. *acetobutylicum* MGC*Δcac15ΔuppΔbukΔctfABΔldh* strain. After selection on Petri plate for clones resistant to erythromycin (40 µg/ml), one colony was cultured for 24 hours in liquid synthetic medium with erythromycin at 40 µg/ml and 100 µl of undiluted culture was plated on RCA with erythromycin at 40 µg/ml and 5-FU at 400 µM. Colonies resistant to both erythromycin and 5-FU were replica plated on both RCA with erythromycin at 40 µg/ml and RCA with thiamphenicol at 50 µg/ml to select clones where 5-FU resistance is also associated with thiamphenicol sensitivity. The genotype of clones resistant to erythromycin and sensitive to thiamphenicol was checked by PCR analysis (with primers PA 0 and PA 5 located outside of the *pta-ack* deletion cassette). The *Δcac15ΔuppΔbuk ΔctfABΔldhΔpta-ack::mls^{R}* strain which have lost pREPΔPA::upp was isolated.

The *Δcac15ΔuppΔbukΔctfABΔldh Δpta-ack::mls^{R}* strain was transformed with pCLF1.1 vector expressing the *Flp.l* gene encoding the Flp recombinase from *S. cerevisiae.* After transformation and selection for resistance to thiamphenicol (50 µg/ml) on Petri plate, one colony was cultured on synthetic liquid medium with thiamphenicol at 50 µg/ml and appropriate dilutions were plated on RCA with thiamphenicol at 50 µg/ml. Thiamphenicol resistant clones were replica plated on both RCA with erythromycin at 40 µg/ml and RCA with thiamphenicol at 50 µg/ml. The genotype of clones with erythromycin sensitivity and thiamphenicol resistance was checked by PCR analysis with primers PA 0 and PA 5. Two successive 24 hours cultures of the *Δcacl5ΔuppΔbukΔctfABΔldhΔpta-ack* strain with erythromycin sensitivity and thiamphenicol resistance were carried out in order to lose pCLF1.1. The *Δcacl5ΔuppΔbukΔctfABΔldhΔpta-ack* strain which has lost pCLF1.1 was isolated according to its sensitivity to both erythromycin and thiamphenicol.

### EXAMPLE 5

### Construction of strains with lower hydrogen production: C. acetobutylicum Δcac1515 Δupp Δbuk ΔctfAB Δldh ΔhydA

To delete the *hydA* gene, the homologous recombination strategy described by Croux & Soucaille (2006) in patent application WO 2008 040387 is used. This strategy allows the insertion of an erythromycin resistance cassette, while deleting most of the genes concerned. The *hydA* deletion cassette in pCons::upp was constructed as follow.

**Table 5 : primers sequences**

| Name | | Primer sequences |
|---|---|---|
| Hyd 1 | SEQ ID N°25 | |
| Hyd 2 | SEQ ID N°26 | |
| Hyd 3 | SEQ ID N°27 | |
| Hyd 4 | SEQ ID N°28 | |
| Hyd 0 | SEQ ID N°29 | CATGTTCTATTGTTACTATGGAAGAGGTAGTAG |
| Hyd 5 | SEQ ID N°30 | GCAGTTATTATAAATGCTGCTACTAGAGC |

Two DNA fragments surrounding *hydA (CAC028)* were PCR amplified with the Pwo polymerase with total DNA from *C*. *acetobutylicum* as template and two specific couples of olignonucleotides. With the couples of primers HYD 1-HYD 2 and HYD 3-HYD 4, 1269 bp and 1317 bp DNA fragments were respectively obtained. Both primers HYD 1 and HYD 4 introduce a BamHI site while primers HYD 2 and HYD 3 have a complementary region which introduces a StuI site. DNA fragments HYD 1-HYD 2 and HYD 3-HYD 4 were joined in a PCR fusion experiment with primers HYD 1 and HYD 4 and the resulting fragment was cloned in pCR4-TOPO-Blunt to yield pTOPO :HYD. At the unique StuI site of pTOPO :HYD, an antibiotic resistance MLS gene with FRT sequences on both sides was introduced from the 1372 bp StuI fragment of pUC 18-FRT-MLS2. The UPP deletion cassette obtained after BamHI digestion of the resulting plasmid was cloned into pCons::upp at the BamHI site to yield the pREPΔHYD::upp plasmid.

The pREPΔHYD::upp plasmid was used to transform by electroporation *C*. *acetobutylicum MGCΔcac15ΔuppΔbukΔqfABôldh* strain. After selection on Petri plate for clones resistant to erythromycin (40 µg/ml), one colony was cultured for 24 hours in liquid synthetic medium with erythromycin at 40 µg/ml and 100 µl of undiluted culture was plated on RCA with erythromycin at 40 µg/ml and 5-FU at 400 µM. Colonies resistant to both erythromycin and 5-FU were replica plated on both RCA with erythromycin at 40 µg/ml and RCA with thiamphenicol at 50 µg/ml to select clones where 5-FU resistance is also associated with thiamphenicol sensitivity. The genotype of clones resistant to erythromycin and sensitive to thiamphenicol was checked by PCR analysis (with primers HYD 0 and HYD 5 located outside of the *hydA* deletion cassette). The *Δcacl5ΔuppΔbukΔctfABΔldhΔhydA::mls^{R}* strain which have lost pREPΔHYD::upp was isolated.

The *Δcac15ΔuppΔbukΔctfABΔldhΔhydA::mls^{R}* strain was transformed with pCLF1.1 vector expressing the *Flpl* gene encoding the Flp recombinase from *S*. *cerevisiae.* After transformation and selection for resistance to thiamphenicol (50 µg/ml) on Petri plate, one colony was cultured on synthetic liquid medium with thiamphenicol at 50 µg/ml and appropriate dilutions were plated on RCA with thiamphenicol at 50 µg/ml. Thiamphenicol resistant clones were replica plated on both RCA with erythromycin at 40 µg/ml and RCA with thiamphenicol at 50 µg/ml. The genotype of clones with erythromycin sensitivity and thiamphenicol resistance was checked by PCR analysis with primers HYD 0 and HYD 5. Two successive 24 hours cultures of the *Δcac15ΔuppΔbukΔctfABΔldhΔhydA* strain with erythromycin sensitivity and thiamphenicol resistance were carried out in order to lose pCLF1.1. The *Δcac15ΔuppΔbukΔctfABΔldhΔhydA* strain which has lost pCLF1.1 was isolated according to its sensitivity to both erythromycin and thiamphenicol.

### EXAMPLE 6

### Batch fermentation of n-butanol producing strains.

Strains were initially analyzed in anaerobic flask cultures in the synthetic medium described by Soni et al (Soni et al, 1987, Appl. Microbiol.Biotechnol. 27:1-5) supplemented with 2.5 g/l of ammonium acetate. An overnight culture at 35°C was used to inoculate a 30 ml culture to an OD600 of 0.05. After incubation of the culture for 3 days at 35°C, glucose, organic acids and solvents were analyzed by HPLC using a Biorad HPX 97H column for the separation and a refractometer for the detection.

Strains with the correct phenotype were subsequently tested under production conditions in 300 ml fermentors (DASGIP) using an anaerobic batch protocol.

For this purpose the fermentor was filled with 250 ml of synthetic medium, sparged with nitrogen for 30 min and inoculated with 25 ml of preculture to an optical density (OD600nm) between 0.05 and 0.1.

The temperature of the culture was maintained constant at 35 °C and the pH was permanently adjusted at 5.5 using an NH₄OH solution. The agitation rate was maintained at 300 rpm during the fermentation.

### EXAMPLE 7

### Continuous fermentation of n-hutanol producing strains.

The best n-butanol producing strain was analyzed in chemostat cultures in the synthetic medium described by Soni et al (Soni et al, 1987, Appl. Microbiol.Biotechnol. 27:1-5). An overnight culture at 35°C was used to inoculate a 300 ml fermentors (DASGIP) using an anaerobic chemostat protocol.

For this purpose the fermentor was filled with 250 ml of synthetic medium, sparged with nitrogen for 30 min and inoculated with 25 ml of preculture to an optical density (OD600nm) between 0.05 and 0.1. After 12 hours of batch culture at 35 °C, pH 5.5 (regulated using an NH₄OH solution) and an agitation rate of 300 rpm, the fermentor was continuously fed with oxygen free synthetic medium at a dilution rate of 0.05 h-1 while the volume was kept constant by sequential removal of fermentated medium. Stability of the culture was followed by products analysis using the HPLC protocol previously described.

### EXAMPLE 8

### Evaluation of n-butanol production strains in batch cultures.

Production strains were evaluated in small flasks. 10% of thawed cultures (typically 3ml) were used to inoculate 30ml of synthetic medium (MSL4). A 15 minutes thermal shock at 80°C was applied to kill any vegetative cells present before the initiation of growth. The cultures were then grown at 37°C for 6 to 7 days. Extra-cellular compounds were quantified by HPLC using the following parameters: Eluent (H2SO4) concentration: 0.25mM; Flow: 0.5ml/min; Temperature: 25°C, Time: 50 minutes.

**Table 6: Synthetic medium composition (MSL4).**

| **Compound** | **Concentration** |
|---|---|
| Glucose | 60g/l |
| KH2PO4 | 0,5g/l |
| K2HPO4 | 0,5g/l |
| MgSO4, 7H2O | 0,2g/l |
| FeS04, 7H2O | 0.01g/l |
| CH3COOH | 2,2g/l |
| Aminobenzoic acid (p) | 8mg/l |
| Biotine | 0,04mg/l |

As can be seen in table 2, upon deletion of the gene coding for the butyrate kinase *(buk)* the maximum butyrate concentration detected in the medium decreases from 3.13 g/l after 2 days of culture in the *C*. *acetobutylicum Δcacl5 Δupp* strain, to 0.43 g/l after 5 days of culture in the *C*. *acetobutylicum Δcac15 Δupp Δbuk::MSLr* strain. Notably, the alcohol/glucose yield (Y_{bu} + Yₑₜ) increases significantly, whereas the acetone/glucose yield (Y_{ac}) decreases significantly.

**Table 7: Solvents yield in % g product/g glucose produced and maximal butyrate concentration in g/l, in batch culture by strains described above. SD denotes the standard deviation; MC denotes the Maximum Concentration in g/l.**

| **Genotype** | **Y_{bu} + Yₑₜ** | **SD** | **Y_{ac}** | **SD** | **Butyrate MC** | |
|---|---|---|---|---|---|---|
| | | | | | **48H** | **120H** |
| *C. acetobutylicum ATCC 824 Δcac15 Δupp* | 23.47 | 0.01 | 9.94 | 0.01 | 3.13 | 1.61 |
| *C. acetobutylicum ATCC824 Δcac15 Δupp Δbuk::MSLr* | 38.07 | 2.23 | 4.64 | 2.68 | 0.29 | 0.43 |

### REFERENCES

Desai RP, Harris LM, Welker NE, Papoutsakis ET. Metabolic flux analysis elucidates the importance of the acid-formation pathways in regulating solvent production by Clostridium acetobutylicum. Metab Eng. 1999,1:206-13.
Green EM, Bennett GN. Genetic manipulation of acid and solvent formation in clostridium acetobutylicum ATCC 824 Biotechnol Bioeng. 1998, 58:215-21.
Green EM, Boynton ZL, Harris LM, Rudolph FB, Papoutsakis ET, Bennett GN. Genetic manipulation of acid formation pathways by gene inactivation in Clostridium acetobutylicum ATCC 824. Microbiology. 1996,142 :2079-86.
Harris LM, Desai RP, Welker NE, Papoutsakis ET. Characterization of recombinant strains of the Clostridium acetobutylicum butyrate kinase inactivation mutant: need for new phenomenological models for solventogenesis and butanol inhibition? Biotechnol Bioeng. 2000,;67:1-11.
Soni B. K., Soucaille P. Goma G. Continuous acetone butanol fermentation : influence of vitamins on the metabolic activity of Clostridium acetobutylicum. Appl. Microbiol. Biotechnol. 1987.27 : 1-5.

## Claims

1. A method for the production of n-butanol by culturing a microorganism of a *Clostridia* species in an appropriate culture medium comprising a source of carbon and recovery of n-butanol from the culture medium, wherein the gene *buk* encoding butyrate kinase, involved in butyrate formation, is deleted in the microorganism, the deletion consisting in the removal of at least 50% of the coding sequence of the *buk* gene.

2. The method according to claim 1 wherein the gene *ptb* encoding phospho-transbutyrylase is furthermore deleted.

3. The method according to claim 1 or 2 wherein the gene *ctfAB* encoding CoA-transferase, involved in acetone formation, is attenuated in the microorganism.

4. The method according to claim 3 wherein the gene *adc* encoding aceto-acetate decarboxylase is furthermore deleted.

5. The method according to any one of claims 1 to 4 wherein in the microorganism, the *ldh* gene is deleted.

6. The method as claimed in any one of claims 1 to 5 wherein in the microorganism, at least one gene involved in acetate formation selected among the following :
• *pta* encoding phospho-transacetylase
• *ack* encoding acetate kinase,
is deleted.

7. The method as claimed in any one of claims 1 to 6, wherein the *hyda* gene is attenuated.

8. The method according to anyone of claims 1 to 7 wherein the microorganism is selected among the group consisting of *C. acetobutylicum. C. beijerinckii, C. saccharoperbutylacetonicum* or *C*. *saccharobutylicum.*

9. The method according to anyone of claims 1 to 8 wherein the culture is continuous and stable.

10. The method according to claim 9 comprising the following steps:
a) Fermentation of the microorganism producing n-butanol
b) Elimination of n-butanol during the fermentation by gas striping.
c) Isolation of n-butanol from the condensate by distillation.

11. The microorganism of a *clostridia* species as defined in any one of claims 1 to 8.

12. The microorganism of claim 11, wherein the gene buk involved in butyrate formation is deleted in the microorganism, the deletion consisting in the removal of at least 50% of the coding sequence of the deleted gene.

13. **The microorganism of claim 12, wherein the gene *ptb* encoding phospho-transbutyrylase is furthermore deleted.**

14. The microorganism of claim 12 **or 13**, wherein at least one gene involved in acetone formation selected among *ctfAB* encoding CoA-transferase and *adc* encoding aceto-acetate decarboxylase is deleted, and/or, the *ldh* gene is deleted, and/or, at least one gene involved in acetate formation selected among *pta* encoding phospho-transacetylase and *ack* encoding acetate kinase is deleted and/or the *hydA* gene is attenuated.

15. The microorganism of one of claims **11** to **14**, selected among the group consisting of *C*. *acetobutylicum, C. beijerinckii, C. saccharoperbutylacetonicum* or *C. saccharobutylicum.*

## Patentansprüche

1. Verfahren zur Herstellung von n-Butanol durch Kultivierung eines Mikroorganismus einer *Clostridia-*Art in einem geeigneten Kulturmedium, welches eine Kohlenstoffquelle umfasst, und Gewinnen von n-Butanol aus dem Kulturmedium, wobei das Gen *buk,* welches die Butyratkinase kodiert, die bei der Butyratbildung beteiligt ist, in dem Mikroorganismus deletiert ist, wobei die Deletion in der Entfernung von mindestens 50 % der kodierenden Sequenz des *buk*-Gens besteht.

2. Verfahren gemäß Anspruch 1, wobei außerdem das Gen *ptb*, welches die Phosphotransbutyrylase kodiert, deletiert ist.

3. Verfahren gemäß Anspruch 1 oder 2, wobei das Gen *ctfAB,* welches die CoA-Transferase kodiert, die bei der Acetonbildung beteiligt ist, in dem Mikroorganismus attenuiert ist.

4. Verfahren gemäß Anspruch 3, wobei außerdem das Gen *adc*, welches die Acetoacetat-Decarboxylase kodiert, deletiert ist.

5. Verfahren gemäß irgendeinem der Ansprüche 1 bis 4, wobei in dem Mikroorganismus das *ldh*-Gen deletiert ist.

6. Verfahren gemäß irgendeinem der Ansprüche 1 bis 5, wobei in dem Mikroorganismus wenigstens ein Gen, welches bei der Acetatbildung beteiligt ist und aus folgenden Genen ausgewählt ist:
• *pta,* welches die Phosphotransacetylase kodiert,
• *ack,* welches die Acetatkinase kodiert,
deletiert ist.

7. Verfahren gemäß irgendeinem der Ansprüche 1 bis 6, wobei das *hydA*-Gen attenuiert ist.

8. Verfahren gemäß irgendeinem der Ansprüche 1 bis 7, wobei der Mikroorganismus ausgewählt ist aus der Gruppe bestehend aus *C*. *acetobutylicum, C. beijerinckii, C. saccharoperbutylacetonicum* oder *C*. *saccharobutylicum.*

9. Verfahren gemäß irgendeinem der Ansprüche 1 bis 8, wobei die Kultur kontinuierlich und stabil ist.

10. Verfahren gemäß Anspruch 9, welches die folgenden Schritte umfasst:
a) Fermentieren des n-Butanol produzierenden Organismus,
b) Entfernen von n-Butanol während der Fermentation durch Gasstrippen,
c) Isolieren von n-Butanol aus dem Kondensat durch Destillation.

11. Mikroorganismus einer *Clostridia-Art,* wie in irgendeinem der Ansprüche 1 bis 8 definiert.

12. Mikroorganismus gemäß Anspruch 11, wobei das Gen *buk,* welches bei der Butyratbildung beteiligt ist, in dem Mikroorganismus deletiert ist, wobei die Deletion in der Entfernung von mindestens 50 % der kodierenden Sequenz des deletierten Gens besteht.

13. Mikroorganismus gemäß Anspruch 12, wobei außerdem das Gen *ptb,* welches die Phosphotransbutyrylase kodiert, deletiert ist.

14. Mikroorganismus gemäß Anspruch 12 oder 13, wobei wenigstens ein Gen, welches bei der Acetonbildung beteiligt ist und ausgewählt ist aus *ctfAB,* welches die CoA-Transferase kodiert, und *adc,* welches die Acetoacetat-Decarboxylase kodiert, deletiert ist, und/oder das *Idh-Gen* deletiert ist, und/oder wenigstens ein Gen, welches bei der Acetatbildung beteiligt ist und auswählt ist aus *pta,* welches die Phosphotransacetylase kodiert, und *ack,* welches die Acetatkinase kodiert, deletiert ist, und/oder das *hydA-Gen* attenuiert ist.

15. Mikroorganismus gemäß einem der Ansprüche 11 bis 14, welcher ausgewählt ist aus der Gruppe bestehend aus *C*. *acetobutylicum, C. beijerinckii, C. saccharoperbutylacetonicum* oder *C*. *saccharobutylicum.*

## Revendications

1. Méthode de production de n-butanol par culture d'un micro-organisme d'une espèce *Clostridia* dans un milieu de culture approprié comprenant une source de carbone et par récupération du n-butanol à partir du milieu de culture, dans laquelle le gène *buk* codant la butyrate kinase, impliqué dans la formation du butyrate, est délété dans le micro-organisme, la délétion consistant en l'élimination d'au moins 50 % de la séquence codante du gène *buk.*

2. Méthode selon la revendication 1, dans laquelle le gène *ptb* codant la phosphotransbutyrylase est en outre délété.

3. Méthode selon la revendication 1 ou 2, dans laquelle le gène *ctfAB* codant la CoA-transférase, impliqué dans la formation de l'acétone, est atténué dans le micro-organisme.

4. Méthode selon la revendication 3, dans laquelle le gène *adc* codant l'acéto-acétate décarboxylase est en outre délété.

5. Méthode selon l'une quelconque des revendications 1 à 4, dans laquelle, dans le micro-organisme, le gène *ldh* est délété.

6. Méthode selon l'une quelconque des revendications 1 ou 5, dans laquelle, dans le micro-organisme, au moins un gène impliqué dans la formation d'acétate choisi parmi les suivants :
- le gène *pta* codant la phospho-transacétylase,
- le gène ack codant l'acétate kinase,
est délété.

7. Méthode selon l'une quelconque des revendications 1 à 6, dans laquelle le gène *hydA* est atténué.

8. Méthode selon l'une quelconque des revendications 1 à 7, dans laquelle le micro-organisme est choisi dans le groupe constitué de *C*. *acetobutylicum, C. beijerinckii, C*. *saccharoperbutylacetonicum* ou *C*. *saccharobutylicum.*

9. Méthode selon l'une quelconque des revendications 1 à 8, dans laquelle la culture est continue et stable.

10. Méthode selon la revendication 9, comprenant les étapes suivantes :
a) fermentation du micro-organisme produisant du n-butanol,
b) élimination du n-butanol pendant la fermentation par entraînement au gaz,
c) isolation du n-butanol à partir du condensat par distillation.

11. Micro-organisme d'une espèce *Clostridia* tel que défini dans l'une quelconque des revendications 1 à 8.

12. Micro-organisme selon la revendication 11, dans lequel le gène *buk* impliqué dans la formation du butyrate est délété dans le micro-organisme, la délétion consistant en l'élimination d'au moins 50 % de la séquence codante du gène délété.

13. Micro-organisme selon la revendication 12, dans lequel le gène *ptb* codant la phospho-trans-butyrase est en outre délété.

14. Micro-organisme selon la revendication 12 ou 13, dans lequel au moins un gène impliqué dans la formation de l'acétone choisi parmi le gène *ctfAB* codant la CoA-transférase et le gène *adc* codant l'acéto-acétate décarboxylase est délété et/ou le gène *ldh* est délété et/ou au moins un gène impliqué dans la formation d'acétate choisi parmi le gène *pta* codant la phospho-transacétylase et le gène *ack* codant l'acétate kinase est délété et/ou le gène *hydA* est atténué.

15. Micro-organisme selon l'une quelconque des revendications 11 à 14, choisi dans le groupe constitué de *C*. *acetobutylicum, C. beijerinckii, C. saccharoperbutylacetonicum* ou *C. saccharobutylicum.*
